# EUROPEAN PATENT APPLICATION

(11) **EP 2 147 912 A1**
(43) Date of publication of application: **27.01.2010**
(21) Application number: 08161000.8
(22) Date of filing: 23.07.2008
(51) Int. Cl.: C07D 215/14, A61K 31/47, A61P 31/18

(54) **Styrylquinolines, their process of preparation and their therapeutic uses**

(71) Applicant: BioAlliance Pharma, 75015 Paris (FR)
(72) Inventor: Soma, Emilienne, 75013 Paris (FR); Thibault, Laurent, 91330 Yerres (FR); Michaut, Mathieu, 67400 Illkirch (FR); Wermuth, Camille, Georges, 67100 Strasbourg (FR); Giethlen, Bruno, 67120 Altorf (FR)
(74) Representative: Domenego, Bertrand

(57) **Abstract**

The present invention concerns new substituted styrylquinolines, their process of preparation and their therapeutic uses as integrase inhibitors and/or for the treatment and/or prevention of HIV.

## Description

A subject of the present invention is quinoline derivatives, in particular endowed with inhibitory properties of Human Immuno-deficiency Virus (HIV) integrase.

It also relates to a synthetic process for these derivatives and their biological uses.

The replication cycle of HIV and other retroviruses involves three major viral enzymes: reverse transcriptase, protease and integrase. Integrase catalyzes the integration of the viral DNA into chromosomal DNA of the host infected cell, it is an essential step for the replication of HIV and other retroviruses. Consequently, an integrase inhibitor constitutes *ipso facto* an accurate candidate for blocking infection by HIV, and possibly an effective therapeutic agent.

Polytherapy targeting reverse transcriptase and/or protease and/or integrase is today the only method to effectively combat the rapid development of the virus. Currently, HIV reverse transcriptase and protease are each targeted by about 10 therapeutic agents. However, integrase is targeted by only one commercialized medication: the Merck's Isentress (raltegravir or MK-0518), approved by the U.S. Food and Drug Administration (FDA) in October 2007. A second integrase inhibitor, Gilead's elvitegravir (GS-9137), is in advanced clinical trials.

The integration of the viral DNA into the chromosomal DNA of the infected cells occurs through a two-steps process: (i) in the "3' processing step", integrase in the cytoplasm of the host cell removes a dinucleotide from 3' end of the viral DNA, while (ii) in the "strand transfer step", integrase in the nucleus catalyzes the insertion of the processed 3' end viral DNA into the host cell DNA.

Integrase inhibitors can be divided into two groups: (i) inhibitors of the 3' processing (referred to as INBIs) and (ii) selective strand transfer inhibitors

(referred to as INSTIs) (Pommier Y, and al. Nat Rev Drug Discov 2005, 4:236-248). INBIs act as docking at the HIV DNA-binding site, preventing 3' processing and strand transfer and INSTIs act as occupying the infected cells DNA-binding site, thus only preventing strand transfer (Johnson AA et al. Mol Pharmacol. 2007 71(3):893-901). Raltegravir and elvitegravir belong to the INSTIs group. However, a limit of these inhibitors is the high rate of virus mutations in treated patients leading to INSTIs resistance.

It is thus a substantial advantage to identify a potent inhibitor of the 3' processing step of integrase with a specific mechanism of action leading to the inhibition of the both steps of integration. Moreover this kind of inhibitors could remain still active on virus bearing mutations which lead to INSTIs resistance.

The inventors have studied quinoline derivatives and have demonstrated anti-integrase properties *in vitro* as well as *in vivo*, these properties being accompanied by significant innocuity. WO 98/45269 discloses phenyl substituted quinolines, in particular styryl quinolines, where the various positions of the quinoleine and phenyl moieties may be optionally substituted.

The inventors have now showed that the presence of a substituent on the 5-position of the quinoline moiety, combined with a 8-OH on the quinoleine core surprisingly leads to substantially increased properties: noteworthy their antiviral efficacy, their stability and their biodisponibility.

Indeed said specific substitutions not only improve the 3' processing inhibition of said quinoline derivatives but surprisingly give also the property to specifically inhibit the strand transfer step at submicromolar concentration, which was not the case of the quinoline derivatives described in WO 98/45269 patent application. These new properties lead to a better antiviral efficacy.

By targeting the two steps of the integration process, said new quinoline derivatives are moreover less sensitive to the emergence of resistance compared to Raltegravir and Elvitegravir which only target the last step of the integration process.

The derivatives according to the invention are **characterized in that** they correspond to the general formula (I) : 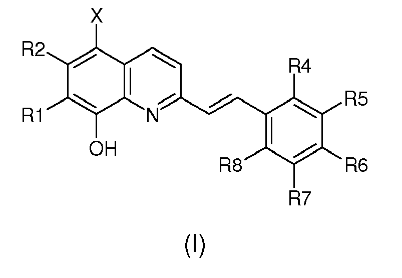 wherein :
- R1, R2, R4, R5, R6, R7, R8 identical or different, independently represent a hydrogen atom or a group chosen from -(CH₂)ₙ-Y or -CH=CH-Y, where Y represents a halogen atom, -OH, -OR, -COH, -COR, -COOH, -COOR, -CONH₂, -CON(Rx,Ry), -CH=NOH, -CO-CH=NOH, -NH₂, -N(Rx,Ry), -NO₂, -PO(OR)₂, -PO(OH)₂, -C(=O)-NH-OH, -SH₂, -SR, -SO₂R, -SO₂NHR, CN,
- X represents a group chosen from -(CH₂)ₙ-Y or -CH=CH-Y, where Y represents a halogen atom, -OH, -OR, -COH, -COR, -COOH, -COOR, -CONH₂, -CON(Rx,Ry), -CH=NOH, -CO-CH=NOH, -NH₂, -N(Rx,Ry), -NO₂, -PO(OR)₂, -PO(OH)₂, -C(=O)-NH-OH, -SH₂, -SR, -SO₂R, -SO₂NHR, CN,
where R represents an alkyl, or an aryl or heterocycle, Rx and Ry, identical or different represent an alkyl, and n is an integer chosen from 0, 1 to 5;
as well as their pharmaceutically acceptable salts, their diastereoisomers and enantiomers.

The present invention also encompasses the following preferred embodiments and any of their combinations:
- X represents a group chosen from a halogen atom, -OH, -OR, -COH, -COR, -COOH, -COOR, -NO₂, CN; more preferably -OH or -NO₂; and/or
- R1 and R2 are H; and/or
- R1, R2, R3, R4, R5, R6, R7 identical or different, independently represent a hydrogen atom or a halogen atom or a group chosen from -OH, -OR, -COH, -COR, -COOH, -COOR, -NO₂, -PO(OR)₂, -PO(OH)₂, -C(=O)-NH-OH, CN; more preferably chosen from a hydrogen atom, halogen atom or a group chosen from -OH, -OR, -COH, -COR, -COOH, -COOR, -NO₂; still more preferably chosen from a hydrogen atom, or a group chosen from -OH, -COR, -COOH, -NO₂, and/or
- two or three of R4, R5, R6, R7, R8 are distinct of H; and/or
- at least one of R4, R5, R6, R7, R8 is OH; and/or
- R6 is OH.

Preferably, the compounds of the invention are chosen from :
- 1-{2-[2(E)-(3-chloro, 4,5-dihydroxyphenyl)-vinyl]-8-hydroxy-quinolin-5-yl}-ethanone;
- 1-{2-[2(E)-(3,4-Dihydroxy-5-methoxy-phenyl)-vinyl]-8-hydroxy-quinolin-5-yl}-ethanone;
- 5-[2(E)-(5-Acetyl-8-hydroxy-quinolin-2-yl)-vinyl]-2-hydroxy-benzoic acid ;
- 8-hydroxy-2-[2(E)- [(3, 4-dihydroxy, 5-methoxyphenyl)ethenyl] 5-quinoline carboxylic acid;
- 1-{2-[2(E)-(2,3-Dihydroxy-4-methoxy-phenyl)-vinyl]-8-hydroxy-quinolin-5-yl}-ethanone;
- 1-{2-[2(E)-(2,4,5-trihydroxyphenyl)-vinyl]-8-hydroxy-quinolin-5-yl}-ethanone;
- 1-{2-[2(E)-(3,4,5-trihydroxyphenyl)-vinyl]-8-hydroxy-quinolin-5-yl}-ethanone ;
- 1-{2-[2(E)-(3,4-dihydroxy, 5-nitrophenyl)-vinyl]-8-hydroxy-quinolin-5-yl}-ethanone ;
- 1-{2-[2(E)-(4-hydroxy, 5-methoxy, 3-nitrophenyl)-vinyl]-8-hydroxy-quinolin-5-yl}-ethanone ;
- 4-[2(E)-(5-Acetyl-8-hydroxy-quinolin-2-yl)-vinyl]-5-nitro-benzoic acid;
- 4-[(E)-2-(5-Acetyl-8-hydroxy-quinolin-2-yl)-vinyl]-3-nitro-benzoic acid methyl ester ;
- 3-[(E)-2-(5-Acetyl-8-hydroxy-quinolin-2-yl)-vinyl]-4-nitro-benzoic acid methyl ester;
- 5-[2(E)-(5-Acetyl-8-hydroxy-quinolin-2-yl)-vinyl]-2-hydroxy-4-nitro-benzoic acid ;
- 1-{2-[2(E)-(3-nitro, 4-hydroxy, 5-methoxyphenyl)-vinyl]-8-hydroxy-quinolin-5-yl}-ethanone ;
- 1-{2-[2(E)-(3,4-dihydroxy, 5-methoxyphenyl)-vinyl]-7-chloro, 8-hydroxy-quinolin-5-yl}-ethanone ;
as well as their pharmaceutically acceptable salts, their diastereoisomers and enantiomers.

Unless specified otherwise, the terms used hereabove or hereafter have the meaning ascribed to them below:
"Halo" or "halogen" refers to fluorine, chlorine, bromine or iodine atom.
"Alkyl" represents an aliphatic-hydrocarbon group which may be straight or branched, having 1 to 20 carbon atoms in the chain unless specified otherwise. Preferred alkyl groups have 1 to 12 carbon atoms, more preferably have 1 to 6 carbon atoms in the chain. Branched means that one or more lower alkyl groups such as methyl, ethyl or propyl are attached to a linear alkyl chain. Exemplary alkyl groups include methyl, ethyl, n-propyl, i-propyl, n-butyl, t-butyl, n-pentyl, 3-pentyl, octyl, nonyl, decyl.
"Aryl" refers to an aromatic monocyclic or multicyclic hydrocarbon ring system of 6 to 14 carbon atoms, preferably of 6 to 10 carbon atoms. Exemplary aryl groups include phenyl, naphthyl, indenyl, phenanthryl, biphenyl.

The terms "heterocycle" or "heterocyclic" refer to a saturated or partially unsaturated non aromatic stable 3 to 14, preferably 5 to 10-membered mono, bi or multicyclic rings, wherein at least one member of the ring is a hetero atom. Typically, heteroatoms include, but are not limited to, oxygen, nitrogen, sulfur, selenium, and phosphorus atoms. Preferable heteroatoms are oxygen, nitrogen and sulfur. Suitable heterocycles are also disclosed in the Handbook of Chemistry and Physics, 76th Edition, CRC Press, Inc., 1995-1996, pages 2-25 to 2-26, the disclosure of which is hereby incorporated by reference.

Preferred non aromatic heterocyclic include, but are not limited to oxetanyl, tetraydrofuranyl, dioxolanyl, tetrahydropyranyl, dioxanyl, pyrrolidinyl, piperidyl, morpholinyl, imidazolidinyl, pyranyl. Preferred aromatic heterocyclic, herein called heteroaryl groups include, but are not limited to, pyridyl, pyridyl-*N*-oxide, pyrimidinyl, pyrrolyl, imidazolinyl, pyrrolinyl, pyrazolinyl, furanyl, thienyl, imidazolyl, triazolyl, tetrazolyl, quinolyl, isoquinolyl, benzoimidazolyl, thiazolyl, pyrazolyl, and benzothiazolyl groups.

"Alkyl", "aryl", "heterocycle" also refers to the corresponding "alkylene", "arylene", "heterocyclene" which are formed by the removal of two hydrogen atoms.

The compounds herein described may have asymmetric centers. Compounds of the present invention containing an asymmetrically substituted atom may be isolated in optically active or racemic forms. It is well-known in the art how to prepare optically active forms, such as by resolution of racemic forms or by synthesis from optically active starting materials. Geometric isomers of double bonds such as olefins and C=N can also be present in the compounds described here, all the stable isomers are contemplated here. All chiral, diastereomeric, racemic forms and all geometric isomeric forms of a compound are intended, unless the stereochemistry or the isomeric form is specifically indicated. All processes used to synthesize the compounds of the present invention are considered as part of the present invention.

The term "substituted" as used herein means that any one or more hydrogens on the designated atom is replaced with a selection from the indicated group, provided that the designated atom's normal valency is not exceeded and that the substitution results in a stable compound.

As used herein, the term "patient" refers to a warm-blooded animal such as a mammal, preferably a human or a human child, which is afflicted with, or has the potential to be afflicted with one or more diseases and conditions described herein.

As used herein, a "therapeutically effective amount" refers to an amount of a compound of the present invention which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

As used herein, the expression "pharmaceutically acceptable" refers to those compounds, materials, compositions, or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, the expression "pharmaceutically acceptable salts" refers to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoechiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418 and P.H. Stahl, C.G. Wermuth, Handbook of Pharmaceutical salts - Properties, Selection and Use, Wiley-VCH, 2002, the disclosures of which are hereby incorporated by reference.

The compounds of the general formula (I) having geometrical and stereoisomers are also a part of the invention.

The invention also relates to a synthetic process for the derivatives defined above. The compounds of formula (I) may be prepared in a number of ways well-known to those skilled in the art. In particular, they may be synthesized by application or adaptation of the process of preparation disclosed in WO 98/45269, or variations thereon as appreciated by the skilled artisan. The appropriate modifications and substitutions will be readily apparent and well-known or readily obtainable from the scientific literature to those skilled in the art.

In particular, such methods can be found in R.C. Larock, Comprehensive Organic Transformations, VCH publishers, 1989

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Synthesis, John Wiley and Sons, 1991; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethyl-formamide; alcohols such as ethanol and methanol and ethers, such as diethyl ether and tetrahydrofuran.

The reactions can take place over a wide range of temperatures. In general, it is convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The process of the invention is **characterized in that** it comprises the reaction of a quinaldine of formula (II) : 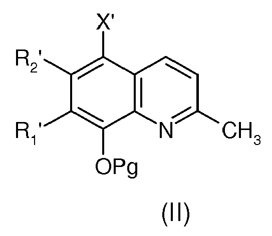 with a compound of formula (III): 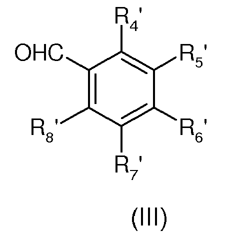 where R1', R2', R4', R5', R6', R7', R8', X' are defined as R1, R2, R4, R5, R6, R7, R8, X above, provided any reactive function present in R1, R2, R4, R5, R6, R7, R8, X may be protected by an appropriate protective group in R1', R2', R4', R5', R6', R7', R8', X' respectively, and where Pg denotes either H or a protective group of the OH function if required,
followed by the deprotection of any protective group present as appropriate.

The coupling may be advantageously conducted in an organic solvent, such as acetic anhydrideand/or a mixture of pyridine/water. The reaction may be carried out at a temperature comprised between the room temperature and the boiling temperature of the reacting mixture.

Generally, OH groups may be protected in the form of acetoxy groups. The deprotection may be conducted by hydrolysis.

The derivatives used as starting products in these syntheses are commercially available or easily accessible by synthesis for a person skilled in the art.

Thus, for example, the derivatives of formula (II) may be synthesized in accordance with Meek et al., J. Chem. Engineering data, 1969, 14, 388-391 or Przystal et al, J.Heterocycl. Chem, 1967, 4, 131-2. As a representative example, the compound of formula (II) where R1=R2=H and X is -COR may be obatined by reacting the corresponding compound of formula (II) where R1=R2=X=H, with a compound of formula X-Hal, where Hal represents a halogen atom.

The compounds of formula (III) are generally commercially available.

If desired, the salts of the compounds of formula (I) may be obtained by adding the appropriate base or acid. For instance, where compounds of formula (I) comprise an acid function the sodium salt may be obtained by adding sodium hydroxide.

The process of the invention may also include the additional step of isolating the obtained product of formula (I). The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well known techniques, such as recrystallization, reprecipitation or the various chromatography techniques, notably column chromatography or preparative thin layer chromatography.

Study of the biological properties of the derivatives of the invention showed an inhibitory activity vis-à-vis HIV integrase *in vitro.* Further experiments have also shown their inhibitory effect on the replication of HIV and the absence of effect on the late phases of the replication of HIV. These results are thus extremely interesting for the treatment of an infection by this virus, especially as the toxicity studies have shown the significant innocuity of these derivatives.

The invention thus relates to pharmaceutical compositions **characterized in that** they contain an effective quantity of at least one derivative as defined above, in combination with pharmaceutically acceptable vehicles.

These compositions are advantageously used in combination with other anti-HIV medicaments, in particular medicaments endowed with an inhibitory effect vis-à-vis the reverse transcriptase and/or protease.

The doses and administration methods are adapted as a function of the single-drug, two-drug or three-drug combination therapy treatment used.

The invention also relates to the use of the derivatives defined above as biological reagents usable in particular for mechanism studies concerning the viral infection.

The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

In general terms, the compounds of this invention may be provided in an aqueous physiological buffer solution containing 0.1 to 10% w/v compound for parenteral and/or oral administration. Typical dose ranges are from 1 µg/kg to 0.1 g/kg of body weight per day; a preferred dose range is from 0.01 mg/kg to 10 mg/kg of body weight per day. A preferred daily dose for adult humans includes 1, 5, 50, 100 and 200 mg, and an equivalent dose in a human child. The preferred dosage of drug to be administered is likely to depend on such variables as the type and extent of progression of the disease or disorder, the overall health status of the particular patient, the relative biological efficacy of the compound selected, and formulation of the compound excipient, and its route of administration.

The compounds of the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical daily dose ranges are from 0.01 to 10 mg/kg of body weight. By way of general guidance, unit doses for humans range from 0.1 mg to 1000 mg per day. Preferably, the unit dose range is from 1 to 500 mg administered one to four times a day, and even more preferably from 1 mg to 300 mg, once a day. Compounds provided herein can be formulated into pharmaceutical compositions by admixture with one or more pharmaceutically acceptable excipients. Such compositions may be prepared for use in oral administration, particularly in the form of tablets or capsules; or parenteral administration, particularly in the form of liquid solutions, suspensions or emulsions; or intranasally, particularly in the form of powders, nasal drops, or aerosols; or dermally, for example, topically or via trans-dermal patches or ocular administration, or intravaginal or intra-uterine administration, particularly in the form of pessaries or by rectal administration.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Preferred formulations include pharmaceutical compositions in which a compound of the present invention is formulated for oral or parenteral administration, or more preferably those in which a compound of the present invention is formulated as a tablet. Preferred tablets contain lactose, cornstarch, magnesium silicate, croscarmellose sodium, povidone, magnesium stearate, or talc in any combination. It is also an aspect of the present disclosure that a compound of the present invention may be incorporated into a food product or a liquid.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

Alternative administrations include also solutions, ointments or other formulations acceptable for ocular administration.

According to a particular aspect, the compound of the invention may be administered by the cutaneous, ocular or inhalation route as disclosed above. These formulations are particularly advantageous as they ensure a local treatment, without associated lymphopenia which may occur with systemic administration routes.

Other features of the invention will become apparent in the course of the following description of exemplary embodiments that are given for illustration of the invention and not intended to be limiting thereof.

### EXAMPLES

### - Example 1 : Synthesis of (E)-1-(2-(3-chloro-4,5-dihydroxystyryl)-8-hydroxyquinolin-5-yl)ethanone, also denoted 1-{2-[2(E)-(3-chloro, 4,5-dihydroxyphenyl)-vinyl]-8-hydroxy-quinolin-5-yl}-ethanone herein (SQE86):

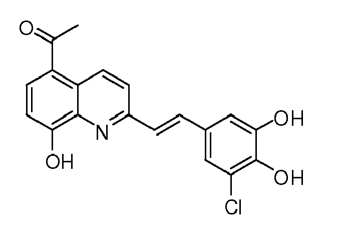

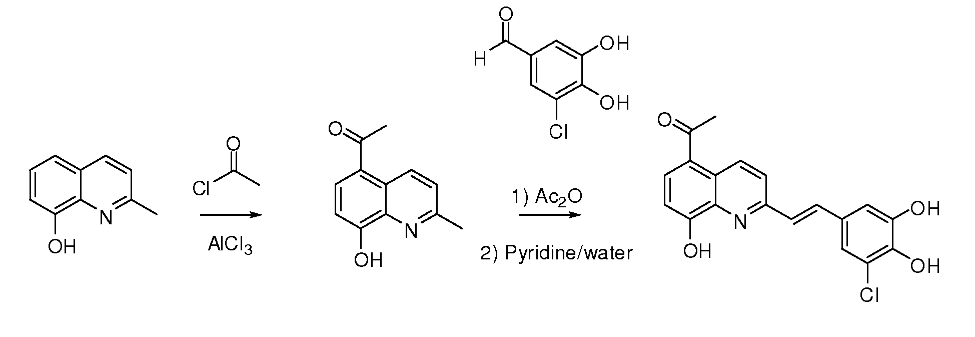

### First step : Preparation of : 1-(8-hydroxy-2-methylquinolin-5-yl)ethanone

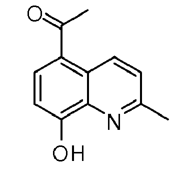

To a solution of commercial 2-methylquinolin-8-ol (4.00 g, 25.13 mmol) in nitrobenzene (10 mL) were added acetyl chloride (1.96 mL, 27.64 mmol) and aluminium chloride (8.38 g, 62.82 mmol). The reaction mixture was heated at 70°C overnight. After the mixture cooled, water and HCl 10% (10 mL) were added with stirring, and reaction mixture was heated at 160°C, collecting nitrobenzene with a Dean-stark trap. After cooling and ethyl acetate washing, mixture was neutralized to pH 6-7 with NaOH. Aqueous phase was extracted with ethyl acetate, then organic layer was washed with brine, dried over MgSO₄ and concentrated under vacuo. Crude product was purified by silica gel chromatography (cyclohexane /ethyl acetate 100:0 to 60/40) to give 1-(8-hydroxy-2-methylquinolin-5-yl)ethanone as a pale yellow powder (3.51 g, 70%).

### Second step: Preparation of (E)-1-(2-(3-chloro-4,5-dihydroxystyryl)-8-hydroxyquinolin-5-yl)ethanone

To a solution of quinoline (0.300 g, 1.49 mmol) in acetic anhydride (10 mL) was added commercial aromatic aldehyde (0.772 g, 4.47 mmol). Mixture was heated in a sealed tube at 160°C for 16h and concentrated under vacuo. Residue was redissolved in a pyridine (10 mL)/ water (5 mL) mixture and heated at 130°C for 3 h. Solvents were evaporated and residue was purified by silica gel chromatography (ethyl acetate/cyclohexane 0:1 to 1:0) to yield (E)-1-(2-(3-chloro-4,5-dihydroxystyryl)-8-hydroxyquinolin-5-yl)ethanone as a yellow solid (0.010 g, 2%).
Fp: 256°-258°C
ESI Mass : m/z 355.92 ([M+H]⁺)

### - Example 2 : Synthesis of (E)-1-(2-(3,4-dihydroxy-5-methoxystyryl)-8-hydroxyquinolin-5-yl)ethanone, also denoted 1-{2-[2(E)-(3,4-Dihydroxy-5-methoxy-phenyl)-vinyl]-8-hydroxy-quinolin-5-yl}-ethanone herein (SQE50) :

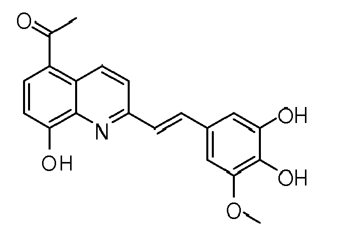

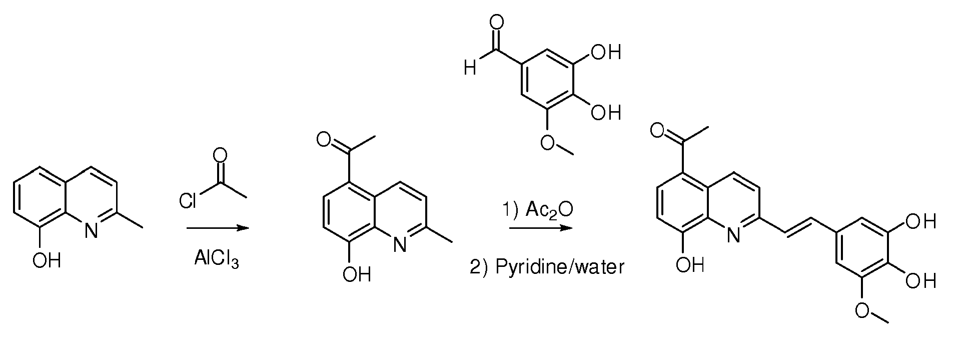

### First step : Preparation of : 1-(8-hydroxy-2-methylquinolin-5-yl)ethanone

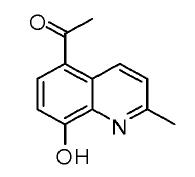

To a solution of commercial 2-methylquinolin-8-ol (4.00 g, 25.13 mmol) in nitrobenzene (10 mL) were added acetyl chloride (1.96 mL, 27.64 mmol) and aluminium chloride (8.38 g, 62.82 mmol). The reaction mixture was heated at 70°C overnight. After the mixture cooled, water and HCl 10% (10 mL) were added with stirring, and reaction mixture was heated at 160°C, collecting nitrobenzene with a Dean-stark trap. After cooling et ethyl acetate washing, mixture was neutralized to pH 6-7 with NaOH. Aqueous phase was extracted with ethyl acetate, then organic layer was washed with brine, dried over MgSO₄ and concentrated under vacuo. Crude product was purified by silica gel chromatography (cyclohexane/ethyl acetate 100:0 to 60/40) to give 1-(8-hydroxy-2-methylquinolin-5-yl)ethanone as pale yellow powder (3.51 g, 70 %).

### Second step: Preparation of (E)-1-(2-(3,4-dihydroxy-5-methoxystyryl)-8-hydroxyquinolin-5-yl)ethanone :

To a solution of quinoline (0.300 g, 1.49 mmol) in acetic anhydride (10 mL) was added commercial aromatic aldehyde (0.752 g, 4.47 mmol). Mixture was heated in a sealed tube at 200°C for 12h and concentrated under vacuo. Residue was redissolved in a pyridine (10 mL)/ water (10 mL) mixture and heated at 110°C for 2h. Solvent were evaporated and residue was triturated with methanol, to give (E)-1-(2-(3,4-dihydroxy-5-methoxystyryl)-8-hydroxyquinolin-5-yl)ethanone as an orange solid (0.090 g, 22%).
Fp: 189 °C-191 °C
ESI Mass : m/z 352.2 ([M+H]⁺)

### - Example 3 : Synthesis of (E)-5-(2-(5-acetyl-8-hydroxyquinolin-2-yl)vinyl)-2-hydroxybenzoic acid, also denoted 5-[2(E)-(5-Acetyl-8-hydroxy-quinolin-2-yl)-vinyl]-2-hydroxy-benzoic acid (SQE 89) :

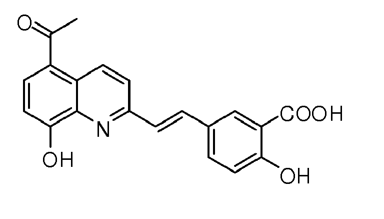

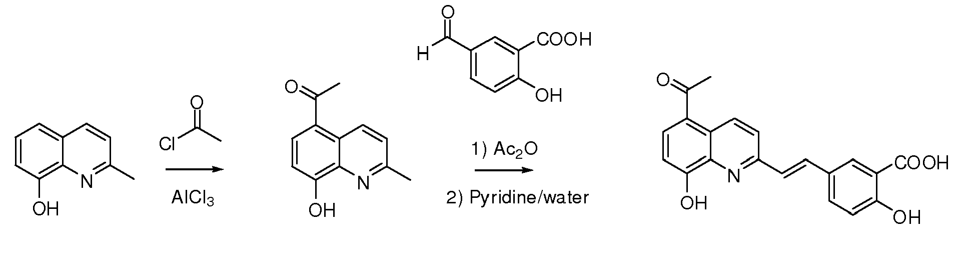

### First step : Preparation of : 1-(8-hydroxy-2-methylquinolin-5-yl)ethanone

To a solution of commercial 2-methylquinolin-8-ol (4.00 g, 25.13 mmol) in nitrobenzene (10 mL) were added acetyl chloride (1.96 mL, 27.64 mmol) and aluminium chloride (8.38 g, 62.82 mmol). The reaction mixture was heated at 70°C overnight. After the mixture cooled, water and HCl 10% (10 mL) were added with stirring, and reaction mixture was heated at 160°C, collecting nitrobenzene with a Dean-stark trap. After cooling and ethyl acetate washing, mixture was neutralized to pH 6-7 with NaOH. Aqueous phase was extracted with ethyl acetate, then organic layer was washed with brine, dried over MgSO₄ and concentrated under vacuo. Crude product was purified by silica gel chromatography (ethyl propionate/ethyl acetate 100:0 to 60/40) to give 1-(8-hydroxy-2-methylquinolin-5-yl)ethanone as a pale yellow powder (3.51 g, 70%).

### Second step: Preparation of (E)-5-(2-(5-acetyl-8-hydroxyquinolin-2-yl)vinyl)-2-hydroxybenzoic acid

To a solution of quinoline (0.300 g, 1.49 mmol) in acetic anhydride (15 mL) was added commercial aromatic aldehyde (0.743 g, 4.47 mmol). Mixture was heated in a sealed tube at 160°C for 12 h and concentrated under vacuo. Residue was redissolved in a pyridine (10 mL)/ water (10 mL) mixture and heated at 110°C for 2h. Solvent were evaporated and residue was triturated with methanol, to give (E)-5-(2-(5-acetyl-8-hydroxyquinolin-2-yl)vinyl)-2-hydroxybenzoic acid (0.217 g, 41.79%) as a dark brown solid.
Fp: 266 °C-268 °C
ESI Mass : m/z 348.40 ([M+H]⁺)

The following compounds were also synthesized by application or adaptation of the procdures above, from the corresponding starting materials:
- 8-hydroxy-2-[2(E)- [(3, 4-dihydroxy, 5-methoxyphenyl)ethenyl] 5-quinoline carboxylic acid: 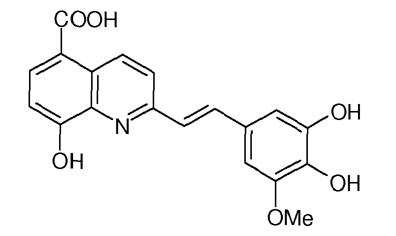
- 1-{2-[2(E)-(2,3-Dihydroxy-4-methoxy-phenyl)-vinyl]-8-hydroxy-quinolin-5-yl}-ethanone : 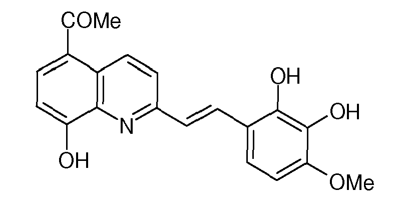
- 1-{2-[2(E)-(2,4,5-trihydroxyphenyl)-vinyl]-8-hydroxy-quinolin-5-yl}-ethanone : 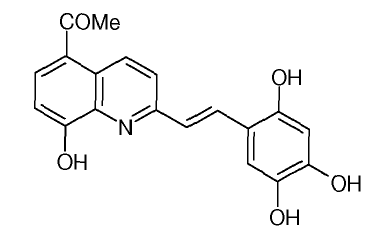
- 1-{2-[2(E)-(3,4,5-trihydroxyphenyl)-vinyl]-8-hydroxy-quinolin-5-yl}-ethanone : 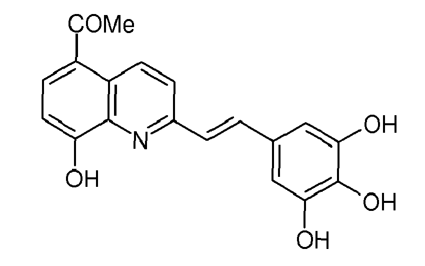
- 1-{2-[2(E)-(3,4-dihydroxy, 5-nitrophenyl)-vinyl]-8-hydroxy-quinolin-5-yl}-ethanone : 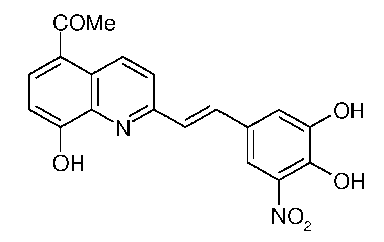
- 1-{2-[2(E)-(4-hydroxy, 5-methoxy, 3-nitrophenyl)-vinyl]-8-hydroxy-quinolin-5-yl}-ethanone : 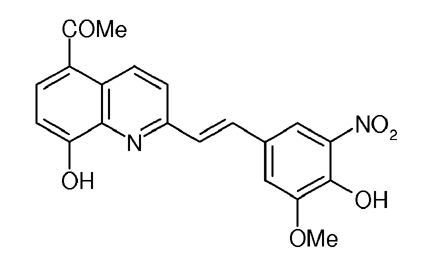
- 4-[2(E)-(5-Acetyl-8-hydroxy-quinolin-2-yl)-vinyl]-5-nitro-benzoic acid: 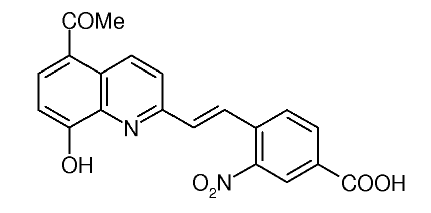
- 4-[(E)-2-(5-Acetyl-8-hydroxy-quinolin-2-yl)-vinyl]-3-nitro-benzoic acid methyl ester 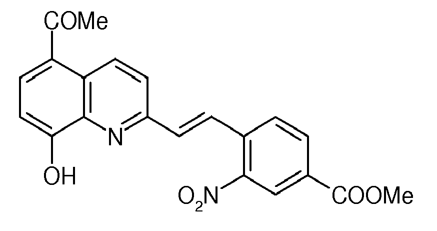
- 3-[(E)-2-(5-Acetyl-8-hydroxy-quinolin-2-yl)-vinyl]-4-nitro-benzoic acid methyl ester: 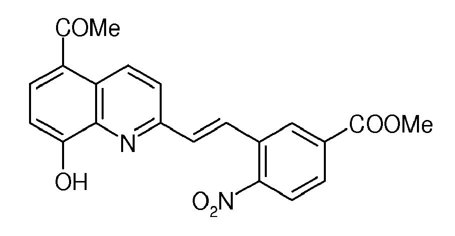
- 5-[2(E)-(5-Acetyl-8-hydroxy-quinolin-2-yl)-vinyl]-2-hydroxy-4-nitro-benzoic acid : 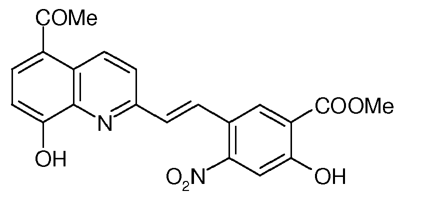
- 1-{2-[2(E)-(3-nitro, 4-hydroxy, 5-methoxyphenyl)-vinyl]-8-hydroxy-quinolin-5-yl}-ethanone : 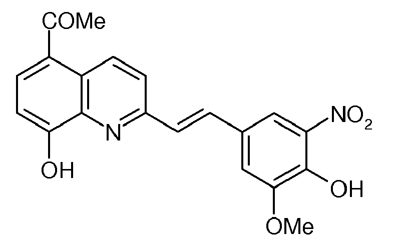
- 1-{2-[2(E)-(3,4-dihydroxy, 5-methoxyphenyl)-vinyl]-7-chloro, 8-hydroxy-quinolin-5-yl}-ethanone :

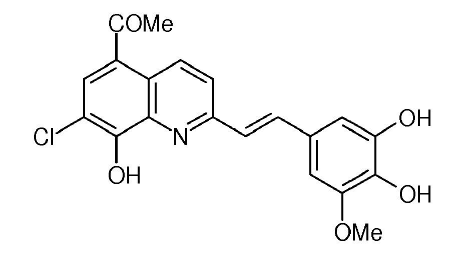

### I.1-Biochemical activity assay

### Integrase Preparation and purification

The pET-15b-IN plasmid contains the cDNA encoding the HBX2 HIV integrase. His-tagged integrase protein was overexpressed in Escherichia coli BL21 (DE3) and purified under native conditions. Briefly, at an OD of 0.8, fusion protein expression was induced in bacterial cultures by the addition of IPTG (1 mM). Cultures were incubated for 3 h at 37°C, after which cells were centrifuged. The cell pellet was resuspended in ice-cold buffer A [20 mM Tris-HCl (pH 8), 1 M NaCl, 4 mM β-mercaptoethanol, and 5 mM imidazole], treated with lysozyme for 1 h on ice, and sonicated. After centrifugation (30 min at 10 000 rpm), the supernatant was filtered (0.45 µm) and incubated for at least 2 h with Ni-NTA agarose beads (Pharmacia). The beads were washed twice with 10 volumes of buffer A, 10 volumes of buffer A with 50 mM imidazole, and 10 volumes of buffer A with 100 mM imidazole. His-tagged integrase was then eluted with buffer A supplemented with 50 µM ZnS04 and 1 M imidazole. The integrase concentration was adjusted to 0.1 mg/mL in buffer A. The fusion protein was cleaved using thrombin and dialyzed overnight against 20 mM Tris-HCl (pH 8), 1 M NaCl, and 4 mM β-mercaptoethanol. After removal of biotinylated thrombin by incubation with streptavidin-agarose magnetic beads (Novagen, Madison, WI), a second dialysis was performed for 2 h against 20 mM Tris-HCl (pH 8), 1 M NaCl, 4 mM β-mercaptoethanol, and 20% (v/v) ethylene glycol. Fractions were aliquoted and rapidly frozen at -80°C.

### Nucleic Acid Substrates

Oligonucleotides U5B (5'-GTGTGGAAAATCTCTAGCAGT-3'), U5B-2 (5'-GTGTGGAAAATCTCTAGCA-3'), U5A (5'-ACTGCTAGAGATTTTCCACAC-3') were purchased from Eurogentec (Liege, Belgium) and further purified on an 18% denaturing acrylamide/urea gel. For processing, strand transfer, 100 pmol of U5B, U5B-2, respectively, were radiolabeled using T4 polynucleotide kinase and 50 µCi of [γ-32P]ATP (3000 Ci/mmol). The T4 kinase was heat inactivated, and unincorporated nucleotides were removed using a Sephadex G-10 column (GE HealthCare). NaCl was added to a final concentration of 0.1 M, and complementary unlabeled strand U5A was added to either U5B or U5B-2. The mixture was heated to 90°C for 3 min, and the DNA was annealed by slow cooling.

### 3' LTR Processing Assays

Processing reaction was performed using U5A-U5B, in buffer containing 20 mM Tris (pH 7.2), 50 mM NaCl, 10 mM DTT, and 10mM MgCl2. The reaction was initiated by addition of substrate DNA (12.5 nM), IN 200nM and the mixture was incubated for up to 2 h at 37°C. The reactions were stopped by phenol/chloroform extraction, and DNA products were precipitated with ethanol. The products were separated in TE containing 7 M urea and electrophoresed on an 18% denaturing acrylamide/urea gel. Gels were analyzed using a STORM Molecular Dynamics phosphorimager and quantified with Image Quant™ 4.1 software.

### Strand Transfer Assays

Processing, strand transfer reactions were performed using U5A-U5B-2, in buffer containing 20 mM Tris (pH 7.2), 50 mM NaCl, 10 mM DTT, 10mM MgCl₂. The reaction was initiated by addition of substrate DNA (12.5 nM), IN 200nM and the mixture was incubated for up to 2 h at 37°C. The reaction was stopped by phenol/chloroform extraction, and DNA products were precipitated with ethanol. The products were separated in TE containing 7 M urea and electrophoresed on an 18% denaturing acrylamide/urea gel. Gels were analyzed using a STORM Molecular Dynamics phosphorimager and quantified with Image Quant™ 4.1 software.

### I.2-Stability assays

### 2 protocols were done.

The first one: Hepes Buffer 0.5M, pH 7.5. 16 µl of drug at 25 mM, in 8 ml of Hepes Buffer. Different time points were done, 0h, 2h, 4h, 24h, and 48h.

The second one: Phosphate buffer 50mM, pH: 7.9.16 µl of drug at 25 mM, in 8ml of Hepes Buffer. Different time points were done, 0h, 2h, 4h, 24h, and 48h.

The analysis was done by a wavelength scan 190 nm to 700 nm. The curves superposition gave the profile of the stability of the drug.

### I.3-Antiviral & viability assays

### Viability assay

The cytotoxicity of compounds was evaluated using un-infected and infected HeLa-P4 cell and CEM leukemia cells. CEM cell were obtained from the American Type Tissue Collection (Rockville, MD).

A serial dilution of drugs is done to evaluate the cytotoxicity concentration and is identified by CC50 (concentration of drug which induces 50% of cytotoxicity)

The HeLa-P4 was cultured in the presence or absence of compounds for 2 days. After this time period, cells were cultivated with MTT for 3 hours, further the medium is removed. And the lysis buffer is incubated for 1 hour, followed by plate reading at 540 nm in a microplate reader.

### Antiviral activity assay on Hela P4 cells

The antiviral activity is determined by infecting HelaP4 cells with a wild type HIV-1 virus (NL 4.3 strains at 3ng) on cells in presence or absence of drugs.

A serial dilution of drugs is done to evaluate the EC50. The effective concentration is the concentration of product at which virus replication is inhibited by 50 percent.

After 48hours incubation, the quantification is done by the evaluation of β-Galactosidase produced by the infected Hela P4 cells. The viral activity is evaluated by colorimetric assay, CPRG, followed by plate reading at 570 nm with a reference of 690 nm. The CPRG test is a colorimetric assay which allows to quantity the β-galactosidase produced by HIV-1 infected indicator cells (the β-Gal gene being under the control of the HIV-1 LTR).

### Antiviral activity assay CEM cells

The activity is determined by infecting CEM cells with a wild type HIV-1 virus (NL 4.3 strains at 3ng) on cells in presence or absence of drugs.

A serial dilution of drugs is done to evaluate the EC50.

The effective concentration is the concentration of product at which virus replication is inhibited by 50 percent.

After 48 hours incubation, the quantification is done by the evaluation of the viral protein p24 with a commercial Elisa Kit. P24 is a protein essential to the replication virus cycle. The quantification of this enzyme is proportional to the amount of virus produced by the infected cells.

### Cross-Resistance assay

Antiviral products targeting the same protein (typically products of the same drug class) may develop mutations that lead to reduced susceptibility to one antiviral product and can result in decreased or loss of susceptibility to other antiviral products in the same drug class. This observation is referred to as cross-resistance. Cross-resistance is not necessarily reciprocal, so it is important to evaluate the activity of our new compounds on viruses containing mutations observed with other drugs of the anti-integrase class.

Resistant mutant viruses of Raltegravir & Elvitegravir were constructed on the NL43 backbone. Mutants viruses were as followed, where the first letter corresponds to the wild type aa, the number corresponds to the position of the aa in the integrase sequence and the second letter corresponds to the mutated aa.
NL4.3 E92Q,
N L4.3 G140S,
NL4.3 Q148H,
NL4.3 N155H,
NL4.3 E92Q+ N155H,
NL4.3 G140S/ Q148H.

The assay to evaluate the activity against resistant viral strains is the same that antiviral assay. Instead of using a Wild-type virus, the virus studied contains mutations in the integrase protein. The results are presented as a fold change corresponding to the ratio between the IC50 obtained for the mutant virus and the IC50 obtained for the wild type virus. More the virus is resistant to the compound more the fold change is elevated. If the mutation has no impact on the activity of the drug, the fold change is around 1.

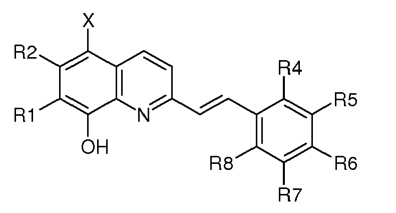

| **Name** | **R1** | **R2** | **X** | **R4** | **R5** | **R6** | **R7** | **R8** | **Processing IC50 (**µ**M)** | **Transfert IC50** | **P4 IC50 (**µ**M)** | **CEM (**µ**M)** | **Cytotoxicity IC50 (**µ**M)** | **Stability** |
|---|---|---|---|---|---|---|---|---|---|---|---|---|---|---|
| SQE50 | H | H | COMe | H | OH | OH | OMe | H | <0.250 | <0.250 | 1.4 | 5 | 7 | +/- |
| SQE56 | H | H | COOH | H | OH | OH | OMe | H | 0,51 | 0,68 | 18 | 60 | >100 | - |
| SQE74 | H | H | COMe | OH | H | OH | OH | H | 0,225 | 0,225 | 11,8 | 0,35 | 11,7 | +/- |
| SQE75 | H | H | COMe | H | OH | OH | OH | H | 0,51 | 0,51 | 9,6 | 24,7 | >100 | ++ |
| SQE79 | H | H | COMe | H | OH | OH | NO₂ | H | 0,225 | 0,225 | 4,6 | 0,4 | 4.2 | + |
| SQE86 | H | H | COMe | H | CI | OH | OH | H | 0,53 | 0,53 | 1 | 0,5 | 15 | + |
| SQE89 | H | H | COMe | H | COOH | OH | H | H | 0,56 | 0,56 | 1 | - | 12,6 | - |
| SQE94 | H | H | COMe | H | NO2 | OH | OMe | H | 0,724 | 0,724 | 1.6 | - | 56 | - |
| SQE96 | H | H | COMe | H | H | COOH | H | NO₂ | 2,3 | 2,3 | 40.0 | - | >100 | - |

As a comparative exemple, the following compound representative for compounds disclosed in WO98/45269 was also tested, as shown below :

| **Name** | **R1** | **R2** | **X** | **R4** | **R5** | **R6** | **R7** | **R8** | **Processing IC50 (**µ**M)** | **Transfert IC50** | **P4 IC50 (**µ**M)** | **CEM (**µ**M)** | **Cytotoxicity IC50 (**µ**M)** | **Stability** |
|---|---|---|---|---|---|---|---|---|---|---|---|---|---|---|
| FZ41 | COOH | H | H | H | OH | OH | OMe | H | 0,7 | 1,7 | 5 | 25 | >100 | - |

It is apparent from the results above that the compounds of the invention are more active than those of the prior art, in particular during the first step (3' processing), and the second step (strand transfert step).

## Claims

1. A compound of general formula (I): 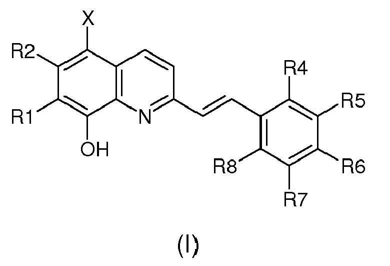 wherein :
- R1, R2, R4, R5, R6, R7, R8 identical or different, independently represent a hydrogen atom or a group chosen from -(CH₂)ₙ-Y or -CH=CH-Y, where Y represents a halogen atom, -OH, -OR, -COH, -COR, -COOH, -COOR, -CONH₂, -CON(Rx,Ry), -CH=NOH, -CO-CH=NOH, -NH₂, -N(Rx,Ry), -NO₂, -PO(OR)₂, -PO(OH)₂, -C(=O)-NH-OH, -SH₂, -SR, -SO₂R, -SO₂NHR, CN,
- X represents a group chosen from -(CH₂)ₙ-Y or -CH=CH-Y, where Y represents a halogen atom, -OH, -OR, -COH, -COR, -COOH, -COOR, -CONH₂, -CON(Rx,Ry), -CH=NOH, -CO-CH=NOH, -NH₂, -N(Rx,Ry), -NO₂, -PO(OR)₂, -PO(OH)₂, -C(=O)-NH-OH, -SH₂, -SR, -SO₂R, -SO₂NHR, CN,
where R represents an alkyl, or an aryl or heterocycle, Rx and Ry, identical or different represent an alkyl, and n is an integer chosen from 0, 1 to 5;
as well as their pharmaceutically acceptable salts, their diastereoisomers and enantiomers.

2. A compound according to claim 1, wherein X represents a group chosen from a halogen atom, -OH, -OR, -COH, -COR, -COOH, -COOR, -NO₂, CN.

3. A compound according to claim 1 or 2, wherein X represents -OH or -NO₂.

4. A compound according to anyone of the preceding claims, wherein R1 and R2 are H.

5. A compound according to anyone of the preceding claims, wherein R1, R2, R3, R4, R5, R6, R7 identical or different, independently represent a hydrogen atom or a halogen atom or a group chosen from -OH, -OR, -COH, -COR, -COOH, -COOR, -NO₂, -PO(OR)₂, -PO(OH)₂, -C(=O)-NH-OH, CN.

6. A compound according to anyone of the preceding claims, wherein R1, R2, R3, R4, R5, R6, R7 identical or different, are chosen from a hydrogen atom, halogen atom or a group chosen from -OH, -OR, -COH, -COR, -COOH, -COOR, -NO₂.

7. A compound according to anyone of the preceding claims, wherein R1, R2, R3, R4, R5, R6, R7, identical or different, are chosen from a hydrogen atom, or a group chosen from -OH, -COR, -COOH, -NO₂.

8. A compound according to anyone of the preceding claims, wherein two or three of R4, R5, R6, R7, R8 are distinct of H.

9. A compound according to anyone of the preceding claims, wherein at least one of R4, R5, R6, R7, R8 is OH.

10. A compound according to anyone of the preceding claims, wherein R6 is OH.

11. A compound according to anyone of the preceding claims chosen from :
- 1-{2-[2(E)-(3-chloro, 4,5-dihydroxyphenyl)-vinyl]-8-hydroxy-quinolin-5-yl}-ethanone;
- 1-{2-[2(E)-(3,4-Dihydroxy-5-methoxy-phenyl)-vinyl]-8-hydroxy-quinolin-5-yl}-ethanone;
- 5-[2(E)-(5-Acetyl-8-hydroxy-quinolin-2-yl)-vinyl]-2-hydroxy-benzoic acid ;
- 8-hydroxy-2-[2(E)- [(3, 4-dihydroxy, 5-methoxyphenyl)ethenyl] 5-quinoline carboxylic acid;
- 1-{2-[2(E)-(2,3-Dihydroxy-4-methoxy-phenyl)-vinyl]-8-hydroxy-quinolin-5-yl}-ethanone;
- 1-{2-[2(E)-(2,4,5-trihydroxyphenyl)-vinyl]-8-hydroxy-quinolin-5-yl}-ethanone;
- 1-{2-[2(E)-(3,4,5-trihydroxyphenyl)-vinyl]-8-hydroxy-quinolin-5-yl}-ethanone ;
- 1-{2-[2(E)-(3,4-dihydroxy, 5-nitrophenyl)-vinyl]-8-hydroxy-quinolin-5-yl}-ethanone ;
- 1-{2-[2(E)-(4-hydroxy, 5-methoxy, 3-nitrophenyl)-vinyl]-8-hydroxy-quinolin-5-yl}-ethanone ;
- 4-[2(E)-(5-Acetyl-8-hydroxy-quinolin-2-yl)-vinyl]-5-nitro-benzoic acid;
- 4-[(E)-2-(5-Acetyl-8-hydroxy-quinolin-2-yl)-vinyl]-3-nitro-benzoic acid methyl ester ;
- 3-[(E)-2-(5-Acetyl-8-hydroxy-quinolin-2-yl)-vinyl]-4-nitro-benzoic acid methyl ester;
- 5-[2(E)-(5-Acetyl-8-hydroxy-quinolin-2-yl)-vinyl]-2-hydroxy-4-nitro-benzoic acid ;
- 1-{2-[2(E)-(3-nitro, 4-hydroxy, 5-methoxyphenyl)-vinyl]-8-hydroxy-quinolin-5-yl}-ethanone ;
- 1-{2-[2(E)-(3,4-dihydroxy, 5-methoxyphenyl)-vinyl]-7-chloro, 8-hydroxy-quinolin-5-yl}-ethanone ;
as well as their pharmaceutically acceptable salts, their diastereoisomers and enantiomers.

12. Process of preparation of a compound according to anyone of claims 1 to 11 comprising the reaction of a corresponding quinaldine of formula (II): 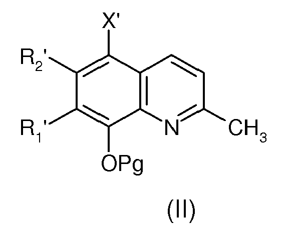 with a compound of formula (III): 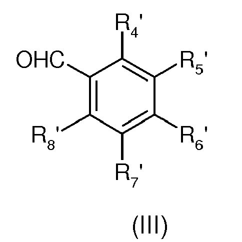
where R1', R2', R4', R5', R6', R7', R8', X' are defined as R1, R2, R4, R5, R6, R7, R8, X in anyone of the preceding claims, provided any reactive function present in R1, R2, R4, R5, R6, R7, R8, X may be protected by an appropriate protective group in R1', R2', R4', R5', R6', R7', R8', X' respectively, and where Pg denotes either H or a protective group of the OH function if required,
followed by the deprotection of any protective group present as appropriate.

13. A pharmaceutical composition comprising a compound of formula (I) according to anyone of claims 1 to 11 with a pharmaceutical acceptable carrier.

14. A compound of formula (I) according to anyone of claims 1 to 11 for the treatment and/or prevention of HIV.

15. A compound of formula (I) according to anyone of claims 1 to 11 for inhibiting integrase.
